**Europäisches Patentamt**

**European Patent Office**

**Office européen des brevets**

(19)

(11) Veröffentlichungsnummer : **0 084 102 B1**

(12) **EUROPÄISCHE PATENTSCHRIFT**

(45) Veröffentlichungstag der Patentschrift :
30.10.85

(21) Anmeldenummer : **82111063.2**

(22) Anmeldetag : **30.11.82**

(51) Int. Cl.⁴ : **G 01 N 33/80, B 01 L 3/00**

(54) **Mikrotiterplatte zur Blutgruppendiagnostik.**

(30) Priorität : **28.12.81 DEU 8137962**

(43) Veröffentlichungstag der Anmeldung :
**27.07.83 Patentblatt 83/30**

(45) Bekanntmachung des Hinweises auf die Patenterteilung : **30.10.85 Patentblatt 85/44**

(84) Benannte Vertragsstaaten :
**AT BE CH DE FR GB IT LI LU NL SE**

(56) Entgegenhaltungen :
**DE-A- 2 152 068**
**DE-A- 2 636 616**
**DE-A- 2 938 009**
**DE-U- 8 029 596**
**Die Akte enthält technische Angaben, die nach dem Eingang der Anmeldung eingereicht wurden und die nicht in dieser Patentschrift enthalten sind.**

(73) Patentinhaber : **Biotest-Serum-Institut GmbH Flughafenstrasse 4**
**D-6000 Frankfurt-Niederrad (DE)**

(72) Erfinder : **Uthemann, Horst, Dr., Dipl.-Chem. Sachenhäuser Landwehrweg 66**
**D-6000 Frankfurt 70 (DE)**
Erfinder : **Schleussner, Hans, Dr., Dipl.-Chem. Nansenring 26**
**D-6000 Frankfurt (DE)**
Erfinder : **Merz, Dieter, Dr. Dipl.-Chem. Gemündener Strasse 34**
**D-6000 Frankfurt 70 (DE)**

(74) Vertreter : **Beil, Walter, Dr. et al**
**BEIL, WOLFF & BEIL Rechtsanwälte Adelonstrasse 58**
**D-6230 Frankfurt am Main 80 (DE)**

**Beschreibung**

Die Erfindung betrifft eine Mikrotiterplatte zur Blutgruppendiagnostik, bestehend aus einer Flachbodenplatte (1) aus einem transparenten, starren Polystyrol, die mit einer Vielzahl von Näpfchen (2) ausgestattet ist.

Derartige Mikrotiterplatten finden ihre Anwendung in der Blutgruppenserologie in Blutbanken, Krankenhäusern und bei Vaterschaftsbestimmungen. Die Durchführung von Blutgruppenbestimmungen nach Mikromethoden wurde bereits in folgenden Druckschriften beschrieben : 1. Crawford, M.N., Gottman, F.E. und C.A. Gottman : Microplate System for Routine Use in Blood Bank Laboratories, Transfusion 10, 258-263 (1970), 2. Parker, J.L., Marcoux, D.A., Hafleigh, E.B., und F.C. Grumet : Modified Microtiter Tray Method for Blood Typing, Transfusion 18, 417-422 (1978), 3. Lapinski, F.J., Crowlex, K.M. Merrit, C.A. und J.B. Henry : Use of Microplate Methods in Paternity Testing, Amer. Soc. Clin. Path, 70, 766-769 (1978), 4. Depew, K.A., Balk, J.W., Meiers, J. und J.B. Schorr : Adaptation of the Microtiter Technique for Use in a Blood Bank Laboratory, AABB, Atlanta, Nov. 1977, 5. Warlow, A. und D. Tills : Micromethods in Blood Group Serology, Vox Sanguinis 35, 354-356 (1978), 6. American Red Cross : Application of Microplates in Regional Blood Centers, National Headquaters, Washington, D.C.

Gegenüber herkömmlichen Methoden, wie der Tüpfelplatten-, Röhrchen-Zentrifugations- oder Slide-Test-Methode besitzt die Mikromethode folgende Vorteile : geringere Kosten, Zeiteinsparung, gute Eignung für Routineanwendung und höhere Empfindlichkeit.

In (2) werden flexible Mikrotiterplatten aus PVC mit V-förmigen Näpfchen, in (1) und (3) feste Mikrotiterplatten aus Polystyrol mit U-förmigen Näpfchen und in (5) sogenannte Terasaki-Platten beschrieben. Die Anwendung umfaßt Bestimmungen im ABO-, im Rh- und in anderen Systemen und schließt gegebenenfalls den Antihumanglobulintest ein. Auch die Durchführung von Serumgegenproben ist beschrieben.

Obgleich die beschriebenen Methoden in Mikrotiterplatten eine Verbesserung der herkömmlichen Methoden darstellen, ist allen gemein, daß flüssige Antiseren entweder unmittelbar vor der Untersuchung in die dafür vorgesehenen Näpfchen eingefüllt oder auf Vorrat eingefüllt und eingefroren werden.

Das Einfüllen der flüssigen Antiseren, ob an Ort und Stelle oder zum Einfrieren, ist relativ zeitraubend und umständlich. Es müssen immer eine Reihe von Gefäßen mit den flüssigen Antiseren verfügbar sein. Darüberhinaus besitzen gewisse Antiseren im flüssigen Zustand nur eine begrenzte Lagerfähigkeit. Und schließlich sind für das Einfüllen der Antiseren Pipetten erforderlich.

Im Zusammenhang mit flüssigen Antiseren wird außerdem in (2) auf Schwierigkeiten wie elektrostatische Aufladung der Platten und dadurch bedingt unkontrolliertes Anhaften von Antiseren an den Wänden, hingewiesen.

In der DE-A-1 26 36 616 werden Reagentien zur immunologischen Bestimmung bzw. Testsysteme gemäß der sogenannten Solid-phase-Technik beschrieben. Dabei macht man sich die seit ca. 20 Jahren bekannte Tatsache zunutze, daß sich einzelne Proteinmoleküle auf irgendwelche Kunststoffoberflächen und andere feste Phasen, wozu auch Polystyrol schlechthin gehört, adhesiv aufbringen lassen unter Bildung einer irreversibel gebundenen, d. h. unlöslichen molekularen Schicht. Bei diesem Testsystem handelt es sich um ein sehr langwieriges und umständliches Verfahren. Bis man überhaupt zu der Monolayer-Schicht kommt, wie in Beispiel 1 beschrieben, muß ein verhältnismäßig großer Aufwand getrieben werden. Außerdem besitzt das Präparat nur eine kurzfristige Haltbarkeit, d. h. nur Stunden oder wenige Tage, und der Test muß unmittelbar durchgeführt werden. In Einzellabors ist dieses Verfahren überhaupt nicht praktikabel und noch viel weniger läßt es sich automatisieren.

Aus dem DE-U 80 29 596 sind Blutgruppenidentitätskarten aus einer mit einer Pigmentlackmattierungsschicht versehenen Polyesterfolie bekannt, die auf ihrer Oberfläche eine trockene Schicht aus Antiserum A und B aufweisen. Derartige Karten werden für den sogenannten Bedside-Test verwendet. Der Bed-side-Test wird jedoch unmittelbar vor der Transfusion beim Patienten vorgenommen, wobei Spender- und Empfängerblut auf ABO-Übereinstimmung verglichen werden, um Transfusionszwischenfälle zu vermeiden. Er gilt nicht als anerkannte Blutgruppenbestimmungsmethode (vgl. Richtlinien zur Blutgruppenbestimmung und Bluttransfusion, Bd. 3, 1. Auflage 1980, Seite 25).

Mikrotiterplatten dienen dagegen dem Zweck der Blutgruppenbestimmung im ABO- und Rh-System, und es können die vorgeschriebene Serumgegenprobe durchgeführt und noch andere Faktoren bestimmt werden.

Aufgabe der Erfindung war es somit, eine Mikrotiterplatte bereitzustellen, mit der Blutgruppenbestimmungen wesentlich vereinfacht und bis zu einem gewissen Grad automatisiert werden können und die eine relativ lange Haltbarkeit bzw. Lagerfähigkeit aufweist.

Diese Aufgabe wird erfindungsgemäß durch eine gattungsgemäße Mikrotiterplatte gelöst, die dadurch gekennzeichnet ist, daß die Näpfchenböden (3) festhaftende trockne Schichten (4) aus im wesentlichen reinen Antiseren aufweisen und die Näpfchen aus einem strahlensterilisierten, proteinbindungsfähigen Polystyrol, wie es für Zellkulturen oberflächenbehandelt ist, bestehen.

Bei der Suche nach geeigneten Materialien für Näpfchenböden von Mikrotiterplatten wurde auch Polystyrol schlechthin getestet, was sich als unbrauchbar erwies, da die aufgetrockneten Antiserenschichten nicht fest auf demselben hafteten. Dies mußte den Fachmann davon abhalten, weiter im Bereich der Polystyrole zu suchen.

Es war deshalb überraschend, daß auf starrem, transparentem, strahlensterilisiertem, proteinbindungsfähigem Polystyrol, wie es für Zellkulturen oberflächenbehandelt ist, Antiseren ohne jegliche haftvermittelnden Zusätze ausgezeichnet haften. Antiseren, die in die Näpfchen eingebracht werden, bilden eine festhaftende Schicht auf den Böden der Näpfchen. Simulierte Transportbedingungen konnten kein Ablösen der Schicht bewirken. Erfindungsgemäß werden somit gebrauchsfertige, lagerfähige, mit getrocknetem Antiserum beschichtete Mikrotiterplatten bereitgestellt, bei denen die Verwendung und das Einfüllen flüssiger Antiseren entfällt und die ohne Aufwand hergestellt und behandhabt werden können. Es wird lediglich ein Tropfen Kochsalzaufschwemmung der zu untersuchenden Blute zugegeben, wobei sich durch leichtes Schütteln die Antiseren lösen. Nach kurzem Zentrifugieren (z. B. 2 Minuten bei 1 000 U/Min.) — ohne vorausgehende Inkubation — wird aufgeschüttelt und ausgewertet.

Als besonders gut geeignete Mikrotiterplatten erwiesen sich Flachbodenplatten mit zylindrischen Näpfchen aus strahlensterilisiertem Polystyrol, wie sie für die Verwendung für Zellkulturen im Costar[R] Kat. Nr. 3596 beschrieben werden. Diese Platten weisen eine hohe Proteinbindungsfähigkeit auf. Auch die zylindrische Ausbildung der Näpfchenböden scheint einen positiven Einfluß auf die Haftfähigkeit der Seren auszuüben, gegenüber U-förmigen oder V-förmigen Böden, wobei jedoch letztere ebenfalls mit Erfolg anzuwenden sind.

Diese Platte besitzt, ähnlich wie bekannte Mikrotiterplatten, eine Vielzahl von längs und quer in einer Ebene nebeneinander angeordneten Näpfchen. Dadurch bietet sich die Möglichkeit vielfältiger Serum und Antiserumkombinationen.

Die erfindungsgemäßen Platten bzw. Näpfchenböden sind mindestens mit Antiseren zur Blutgruppenbestimmung im ABO- und im Rh-System beschichtet. Die Antiseren für das ABO-System zeichnen sich durch hohe Avidität aus, während für das Rh-System chemisch modifizierte Antiseren (z. B. Anti-D oder Anti-CDE) verwendet werden, die ebenfalls im Kochsalzmilieu rasch agglutinieren.

Chemisch modifizierte IgG-Moleküle, bei denen Disulfidbrücken in der Hinge-Region teilweise reduktiv aufgespalten und alkyliert worden sind, zeichnen sich durch hohe Flexibilität aus, weshalb bei Antiseren auf der Basis der modifizierten Moleküle auf den üblichen Zusatz von Supplement (z. B. Albumin, Dextran, Gelatine, AB-Serum u. a.) verzichtet werden kann. Die chemische Modifizierung überführt inkomplette Antikörper in Agglutinine, die ähnliche Eigenschaften wie ABO-Testseren haben. So werden mit modifiziertem IgG der Spezifität Anti-D D(Rh$_o$)-positive Erythrozyten in Kochsalzlösung oder in verträglichem Serum bzw. Plasma agglutiniert. In Pirofsky B. und Cordova M.S. « Bivalent nature of incomplete anti-D (Rh$_o$) » Nature 197, S. 392-393 (1963) ; Roman D., Tilley C.A., Crookstone N.C., Falk R.E. and Dorington K.J. « Conversion of incomplete antibodies to direct agglutinins by mild reduction. Evidence for segmental flexibility within the F$_c$ fragment of immunglobuline G » Proc. Natl. Akad. Sci. USA, 74, S. 2531-2535 (1977) wird dieses Anti-D-Reagens beschrieben. Bisher wurden der Objektträger-Schnelltest, der Objektträger-Inkubationstest und der Röhrchenzentrifugiertest als Anwendungsmöglichkeiten vorgeschlagen.

Überraschenderweise wurde gefunden, daß das Anti-D-Reagens, auf den Näpfchenböden aus dem oberflächenbehandelten Polystyrol angetrocknet, ohne vorher mit Wasser aufgelöst werden zu müssen, beim Verrühren mit D(Rh$_o$)-positivem Blut nach kurzer Reaktionszeit (ca 1. Minute) in ihrer Stärke dem ABO-System vergleichbare Agglutinationsbilder liefert. Weil weder das Reagens selbst Supplemente enthält, noch für das Antrocknen auf dem Näpfchenboden hochpolymere, eine Haftung vermittelnde Zusätze erforderlich sind, kann auf eine Kontrolle verzichtet werden. Es ist nicht zu erwarten und wurde bisher auch nicht beschrieben, daß das Anti-D-Reagens mit seiner niedrigen Proteinkonzentration, die der Konzentration von normalem humanen Serum entspricht, zu falsch positiven Reaktionen mit IgG- und/oder komplementbeladenen Erythrozyten bei Autoimmunerkrankungen oder der Erythroblastose des Neugeborenen führt, die aber häufig mit inkompletten Anti-Rh-Seren, die Rinderalbumin oder andere hochmolekulare Substanzen als Verstärkermedium enthalten, beobachtet werden.

Außer mit dem ABO- und Rh-System, z. B. Anti-D und Anti-CDE, können die Platten zweckmäßigerweise noch mit Anti-AB, AB-Serum, Anti-Kell und anderen Systemen beschichtet sein.

Die Platte sollte deshalb eine ausreichende Anzahl an Näpfchen aufweisen, um Beschichtungen mit allen gewünschten Systemen vornehmen zu können.

Es sollten also mindestens 7 bis 8 Näpfchen quer nebeneinander angeordnet sein. Handelsübliche Platten weisen 96 Näpfchen auf, d. h. 8 nebeneinander in Querrichtung und 12 nebeneinander in Längsrichtung angeordnete Näpfchenreihen.

Um Verwechslungen zu vermeiden, können die Antiseren-Schichten unterschiedlich gefärbt sein.

Die gebrauchsfertigen Platten sind einzeln in Aluminiumtüten eingeschweißt und bei 2 bis 8 °C zu lagern.

Die Erfindung wird anhand schematischer Zeichnungen näher erläutert.

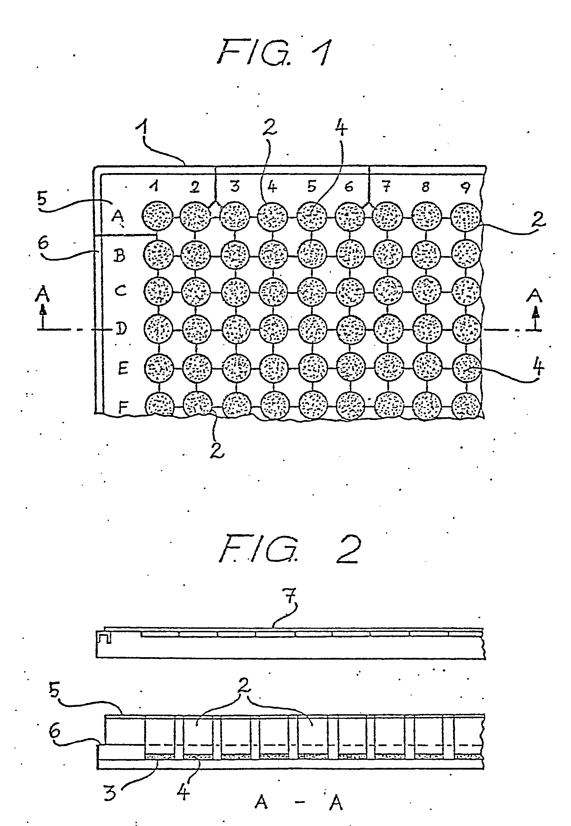

Fig. 1 zeigt eine Draufsicht auf eine Flachbodenplatte (1) mit einer Vielzahl aneinanderhängender zylindrischer Näpfchen (2), deren Böden mit einer Antiserumschicht (4) versehen ist. In der hier gezeigten Ausführungsform erstreckt sich um die ganze Näpfchenanordnung ein Rahmen (5), der mit einem Absatz (6) versehen ist, auf den ein Deckel aufgelegt werden kann. Zur Bezeichnung bzw. Identifizierung der einzelnen Näpfchen sind die längsverlaufenden Näpfchenreihen mit A, B, C usw. und die querverlaufenden Näpfchenreihen mit 1, 2, 3 usw. bezeichnet.

Fig. 2 zeigt einen Schnitt A-A durch die Fig. 1, worin die zylindrischen Näpfchen (2) mit Böden (3) und Antiserumschicht (4) zu sehen sind, die von Rahmen (5) mit Absatz (6) umgeben sind, auf den der Deckel (7) aufgesetzt werden kann.

### Patentansprüche

1. Mikrotiterplatte zur Blutgruppendiagnostik, bestehend aus einer Flachbodenplatte (1) aus einem transparenten, starren Polystyrol, die mit einer Vielzahl von Näpfchen (2) ausgestattet ist, dadurch gekennzeichnet, daß die Näpfchenböden (3) festhaftende trockene Schichten (4) aus im wesentlichen reinen Antiseren aufweisen und die Näpfchen aus einem strahlensterilisierten, proteinbindungsfähigen Polystyrol, wie es für Zellkulturen oberflächenbehandelt ist, bestehen.

2. Platte nach Anspruch 1, dadurch gekennzeichnet, daß die Näpfchenböden (3) zylindrisch ausgebildet sind.

3. Platte nach einem der vorstehenden Ansprüche, dadurch gekennzeichnet, daß der Boden (3) von mindestens je einem Näpfchen mit einer Antiserum A-, Antiserum B-, Antiserum AB-, AB-Serum-, IgG der Spezifität Anti-D- und Anti-CDE-Schicht (4) versehen ist.

4. Platte nach einem der vorstehenden Ansprüche, dadurch gekennzeichnet, daß die Schichten (4) unterschiedlicher Antiseren mit unterschiedlicher Färbung versehen sind.

### Claims

1. Microtiter plate for blood typing consisting of a flat-bottomed plate (1) made of a transparent rigid polystyrene with a number of wells (2), characterized in that the bottoms (3) of the wells have tightly adhering dry layers (4) made of essentially pure antisera and the wells are made of a radiation-sterilized polystyrene of the kind being surface treated for cell cultures and that is able to bind protein.

2. Plate as in Claim 1, characterized in that the bottoms (3) of the wells are cylindrical.

3. Plate as in one of the preceding claims, characterized in that the bottom (3) of at least one of the wells is supplied with a layer (4) of antiserum A, antiserum B, antiserum AB, AB serum, IgG specific to anti-D and to anti-CDE.

4. Plate as in one of the preceding claims, characterized in that the layers (4) of different antisera have different coloring.

### Revendications

1. Plaque de microtitrage pour le diagnostic du groupe sanguin, constituée d'une plaque à fond plat (1) faite d'un polystyrène rigide et transparent, qui est pourvue d'un grand nombre de petits godets (2), caractérisée en ce que le fond des godets (3) présente des couches sèches adhérentes (4) faites pour l'essentiel d'antisera purs, et en ce que les godets se composent d'un polystyrène apte aux liaisons protéiques, stérilisé aux rayonnements, tel que traité en surface pour les cultures cellulaires.

2. Plaque selon la revendication 1, caractérisée en ce que les fonds de godets (3) ont une forme cylindrique.

3. Plaque selon l'une des revendications précédentes, caractérisée en ce que le fond (3) d'au moins chaque petit godet est muni d'une couche d'antisérum A, d'antisérum B, d'antisérum AB, de sérum AB, d'IgG de spécificité anti-D et d'anti-CDE (4).

4. Plaque selon l'une des revendications précédentes, caractérisée en ce que les couches (4) d'antisera différents sont munies d'une coloration différente.

## FIG. 1

## FIG. 2

A - A